(19) Europäisches Patentamt
European Patent Office
Office européen des brevets

(11) Publication number: **0 275 704**
**A1**

## (12) EUROPEAN PATENT APPLICATION

(21) Application number: **87311390.6**

(22) Date of filing: **23.12.87**

(51) Int. Cl.[4]: **C07D 405/06**, C07D 409/06, A01N 43/64

The title of the invention has been amended (Guidelines for Examination in the EPO, A-III, 7.3).

(30) Priority: **23.01.87 GB 8701515**

(43) Date of publication of application: **27.07.88 Bulletin 88/30**

(84) Designated Contracting States: **AT BE CH DE ES FR GB GR IT LI LU NL SE**

(71) Applicant: **IMPERIAL CHEMICAL INDUSTRIES PLC**
**Imperial Chemical House Millbank**
**London SW1P 3JF(GB)**

(72) Inventor: **Elliott, Raymond**
**14 Huntington Close Lower Earley**
**Nr Reading Berkshire(GB)**
Inventor: **Griffin, David Alan**
**10 Chivers Drive**
**Wokingham Berkshire(GB)**
Inventor: **Gairns, Raymond Stevenson**
**10 Windsor Avenue**
**Whitefield Manchester, M25 6BA(GB)**
Inventor: **Hutchings, Michael Gordon**
**11 Belvadere Court St. Anne's Road**
**Prestwich Manchester(GB)**

(74) Representative: **Ricks, Michael James et al**
**Imperial Chemical Industries PLC Legal Department: Patents PO Box 6 Bessemer Road**
**Welwyn Garden City Herts, AL7 1HD(GB)**

# (54) 1,2,4-Triazole derivatives.

(57) A thiophene, furan or tetrahydrofuran triazolyl derivative having the general formula (I) or (II) :

EP 0 275 704 A1

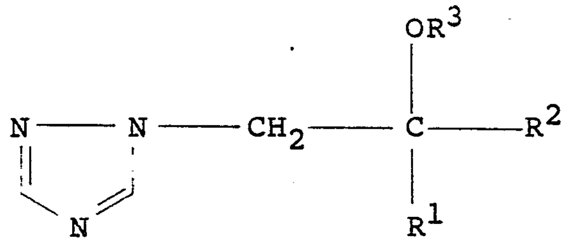

(I)

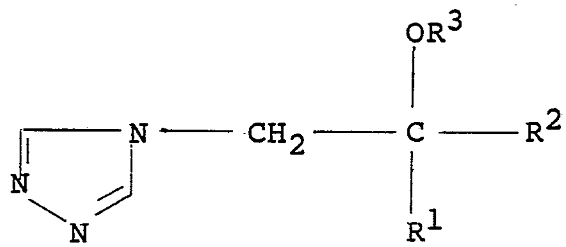

(II)

and stereoisomers thereof, wherein $R^1$ is tertiary butyl, which may be optionally substituted with one or more halogen atoms; $R^2$ is the group :

$-(CH_2)_n-Z$

or $-CH=CH-X$

or $-C\equiv C-Z$

where n is an integer from 0 to 3 and Z is an optionally substituted thiophene, furan, or tetrahydrofuran ring; and X is an optionally substituted thiophene or tetrahydrofuran ring; and $R^3$ is hydrogen or an alkyl group containing from 1 to 4 carbon atoms; and salts, esters and metal complexes of the compound of formula (I) and (II) wherein $R^3$ is hydrogen.

# HETEROCYCLIC COMPOUNDS

This invention relates to heterocyclic compounds, and in particular to thiophene, furan and tetrahydrofuran triazolyl derivatives, useful as plant growth regulating agents, to processes for preparing them, to compositions containing them and to methods of regulating plant growth using them.

According to the present invention there is provided a thiophene, furan or tetrahydrofuran triazolyl derivative having the general formula (I) or (II) :

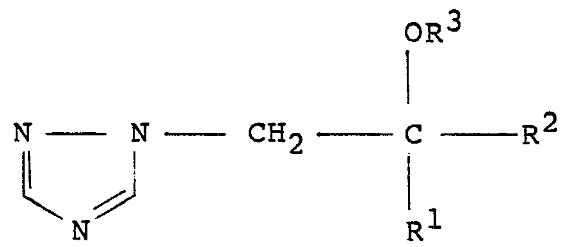

(I)

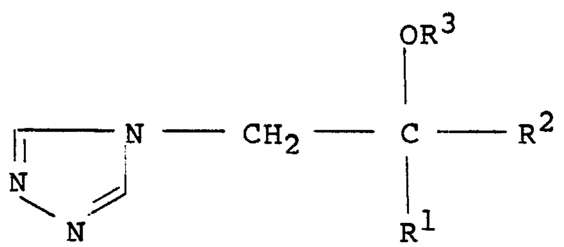

(II)

and stereoisomers thereof, wherein $R^1$ is tertiary butyl, which may be optionally substituted with one or more halogen atoms: $R^2$ is the group

$-(CH_2)_n-Z$

or $-CH=CH-X$

or $-C\equiv C-Z$

where n is an integer from 0 to 3 and Z is an optionally substituted thiophene, furan or tetrahydrofuran and X is an optionally substituted thiophene or tetrahydrofuran ring; and $R^3$ is hydrogen or an alkyl group containing from 1 to 4 carbon atoms; and salts, esters and metal complexes of the compound of formula (I) or (II) wherein $R^3$ is hydrogen.

The compounds of the invention may contain chiral centres. Such compounds are generally obtained in the form of racemic mixtures. However, these and other mixtures can be separated into the individual isomers by methods known in the art, and this invention embraces such isomers.

$R^1$ is preferably unsubstituted tertiary butyl or monohalo-tertiary-butyl, for example chloro-tertiary-butyl or fluoro-tertiary-butyl.

The optionally substituted thiophene, furan or tetrahydrofuran ring, Z, present in the group $R^2$, may be an optionally substituted thiophen-2-yl, thiophen-3-yl, furan-2-yl or furan-3-yl, tetrahydrofuran-2-yl or tetrahydrofuran-3-yl group. The optionally substituted thiophene or tetrahydrofuran ring, X, present in the group $R^2$, may be an optionally substituted thiophen-2-yl, thiophen-3-yl, tetrahydrofuran-2-yl or tetrahydrofurn-3-yl. As optional substituents, there may be mentioned one or more of: alkyl containing from 1 to 6 carbon atoms, preferably from 1 to 4 carbon atoms; cycloalkyl containing from 3 to 6 carbon atoms: alkylcycloalkyl containing a total of from 4 to 6 carbon atoms; or cycloalkylalkyl containing a total of from 4 to 6 carbon atoms; alkenyl containing from 2 to 6 carbon atoms; alkynyl containing from 2 to 6 carbon atoms; and halogen, for example, chlorine, bromine or fluorine.

When $R^2$ is the group $-(CH_2)_n-Z$, n is preferably 0, 1 or 2.

Preferred groups $R^3$ are hydrogen and methyl. Hydrogen is especially preferred.

The present invention includes salts, esters and metal complexes of the compounds of formula (I) or (II) wherein $R^3$ is hydrogen. As examples of esters there may be mentioned for example acetates or benzoates. As examples of salts there may be mentioned for example toluene sulphonate salts, dodecylbenzene sulphonate salts, hydrochloride salts, hydrobromide salts and orthophosphate salts. Without

limitation of the generality of the above statement, the present invention also includes any compound which breaks down in agrochemical use to a compound of formula (I) or formula (II).

Examples of the compounds of the invention are presented in Table I in which the values for $R^1$, $R^2$ and $R^3$ in the general formula (I) or (II) above are as indicated.

TABLE I

| COMPOUND NO. | GENERAL FORMULA | $R^1$ | $R^2$ | $R^3$ | MELTING POINT (°C) |
|---|---|---|---|---|---|
| 1 | (I) | tBu* | 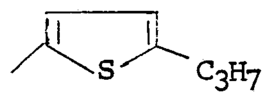 | H | gum |
| 2 | (I) | tBu | 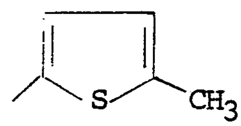 | H | 97-100 |
| 3 | (I) | tBu | 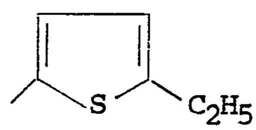 | H | 67-69 |
| 4 | (I) | tBu | 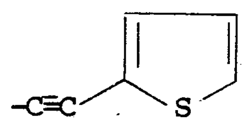 | H | 110-114 |
| 5 | (I) | tBu | 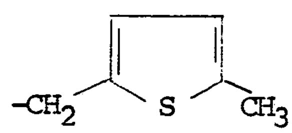 | H | 91-94 |
| 6 | (I) | tBu | 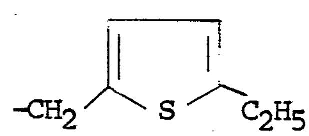 | H | 75-77 |

* tBu represents the tertiary butyl group

TABLE I (CONT/D)

| COMPOUND NO. | GENERAL FORMULA | $R^1$ | $R^2$ | $R^3$ | MELTING POINT (°C) |
|---|---|---|---|---|---|
| 7 | (I) | tBu | 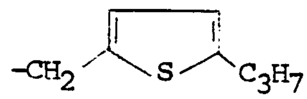 | H | 46-48 |
| 8 | (I) | tBu | 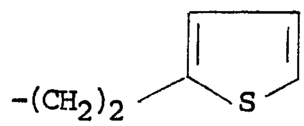 | H | gum |
| 9 | (I) | tBu | 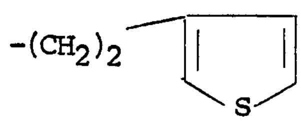 | H | gum |
| 10 | (I) | tBu | 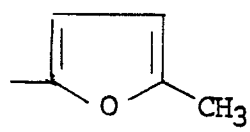 | H | 52-56 |
| 11 | (I) | tBu | 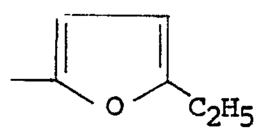 | H | 70-72 |
| 12 | (II) | tBu | 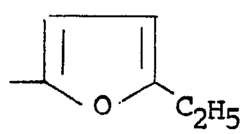 | H | 105-108 |
| 13 | (I) | tBu | 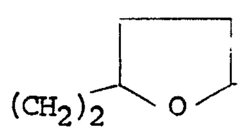 | H | gum |

The invention also includes furan and tetrahydrofuran derivatives corresponding to the thiophene derivatives (Compound Nos. 1 to 9) and thiophene derivatives corresponding to the furan and tetrahydrofuran derivatives (Compound Nos. 10 to 13).

Compounds of general formula (I) above wherein $R^3$ is hydrogen and $R^1$ and $R^2$ are as defined may be prepared by reacting a compound of general formula (IIIa) or (IIIb) :

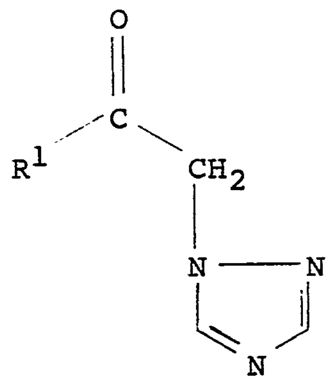

(IIIa)

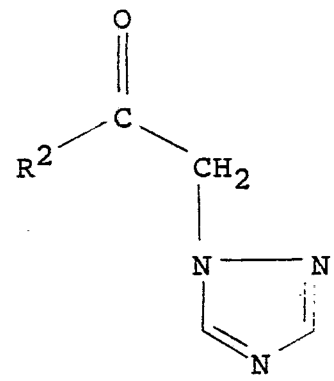

(IIIb)

with an organometallic compound which may be represented by the general formula (IVa) or (IVb) respectively :

$R^1M$ (IVa)

$R^2M$ (IVb)

where M is a suitable metal, for example lithium, magnesium, titanium or zirconium.

The reaction conveniently takes place in a solvent such as diethylether, tetrahydrofuran or dichloromethane at -80°C to +80°C in an inert atmosphere. The product is obtained by quenching with a proton donor. When M is magnesium, the organometallic compound is more specifically $R^2$-Mg-halogen. When M is titanium, the organometallic compound is more specifically $R^2$-Ti(O-alkyl)$_3$. When M is zirconium, the organometallic compound is more specifically $R^2$-Zr-(O-alkyl)$_3$.

The compounds of general formula (I) or (II) wherein $R^3$ is hydrogen may also be prepared by reacting a compound of general formula (V) or (VI), wherein $R^1$ and $R^2$ are as defined and Hal is a halogen atom (preferably a chlorine or bromine atom) with 1,2,4-triazole either in the presence of an acid-binding agent (for example potassium carbonate) or in the form of one of its alkali metal salts in a convenient solvent.

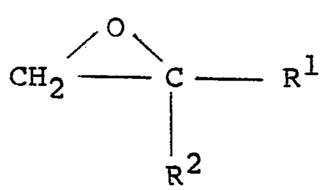

(V)

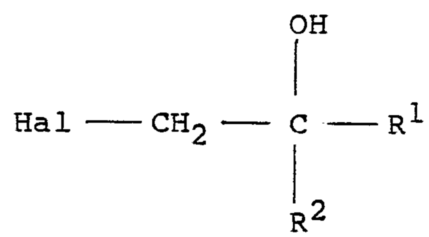

(VI)

Suitably the compound of general formula (V) or (VI) is reacted at 20-100°C with the sodium salt of 1,2,4-triazole in a convenient solvent such as acetonitrile, methanol, ethanol or dimethylformamide. The sodium salt of 1,2,4-triazole can typically be prepared by adding either sodium hydride or sodium methoxide to 1,2,4-triazole. The product can be isolated by pouring the reaction mixture into water and extracting with a suitable solvent, for example diethyl ether, ethyl acetate or dichloromethane.

We have found that reaction of the compound of general formula (V) or (VI) with 1,2,4-triazole in the presence of an acid binding compound such as potassium carbonate in a solvent such as dimethylformamide generally gives a mixture in which the 1-substituted triazole predominates and in which the 4-substituted triazole is present as a minor component. The mixture may be used used without isolating the position isomers, but, if desired, the isomers may be readily separated, for example by fractional crystallisation, chromatography or vacuum distillation. We have found conversely that reaction of the compound of general formula (V) or (VI) with an alkali metal salt of 1,2,4-triazole generally produces essentially the 1-substituted triazole.

Compounds of general formula (I) wherein $R^3$ is hydrogen and $R^2$ is -$CH_2$-Z may also be prepared by

reacting a compound of general formula (VII) :

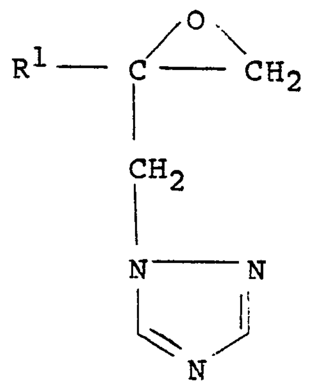

(VII)

with an organometallic compound of general formula Z-M under the conditions indicated previously for the reaction of compounds (IIIa) and (IVb).

The ether (wherein $R^3$ is alkyl) and esters of the invention may be made from the corresponding hydroxy compound by reaction with the appropriate halide, acid chloride or acid anhydride in the presence of a suitable base or with the appropriate acid or metal complexing agents.

The compounds of general formula (V) can be prepared by reacting the appropriate ketone, $R^1COR^2$ - (VIII), with dimethylsulphonium methylide (JACS 1962, 84 3782) or dimethylsulphoxonium methylide (JACS 1965, 87, 1353) using methods set out in the literature.

Compounds of general formula (V) and (VI) may also be prepared by reacting a compound of general formula (IXa) or (IXb) :

Hal—$CH_2$—CO—$R^1$ (IXa)

Hal—$CH_2$—CO—$R^2$ (IXb)

with an organometallic compound of general formula (IVb) or (IVa) respectively.

Olefinic compounds wherein $R^2$ is the group -CH=CH-X may be made by the reduction of the corresponding acetylenic compound wherein $R^2$ is -C≡C-Z. Suitable reducing agents include hydrogen in the presence of a suitable catalyst such as palladium on a support such as carbon: or a metal hydride reducing agent such as lithium aluminium hydride, "Red-Al" (sodium bis [2-methoxyethoxy] aluminium hydride) or sodium borohydride·palladium (II) chloride in a suitable solvent such as ether or tetrahydrofuran.

Similarly, compounds of formula (I) or (II) wherein $R^2$ is a group :

$-(CH_2)_2-Z$

may be made by reduction of the corresponding olefinic compound wherein $R^2$ is the group -CH=CH-X, or by the complete reduction of the corresponding acetylenic alcohol, -C≡C-Z. Suitable reducing agents include hydrogen in the presence of a suitable catalyst such as palladium on a support such as carbon and in a suitable solvent such as methanol, ethanol or acetic acid.

The plant growth regulating effects of the compounds are manifested as, for example, by a stunting or dwarfing effect on the vegetative growth of woody and herbaceous mono-and di-cotyledonous plants. Such stunting or dwarfing may be useful, for example, in peanuts, cereals such as wheat, barley and rice, oil seed rape, field beans, sunflowers, potatoes and soya bean where reduction in stem height, with or without further advantageous effects such as stem strengthening, thickening and shortening, internode shortening, increased buttress root formation and more erect stem and leaf orientation, may reduce the risk of lodging and may also permit increased amounts of fertiliser to be applied. The stunting on woody species is useful in controlling the growth of undergrowth under power lines etc. Compounds which induce stunting or dwarfing may also be useful in modifying the stem growth of sugar cane thereby increasing the concentration of sugar in the cane at harvest; in sugar cane, the flowering and ripening may be controllable by applying the compounds. Stunting of peanuts can assist in harvesting. Growth retardation of grasses can help maintenance of grass swards. Examples of suitable grasses are Stenotaphrum secundatum (St. Augustine grass), Cynosurus cristatus, Lolium multiflorum and perenne Agrostis tenuis, Cynodon dactylon - (Bermuda grass), Dactylis glomerata, Festuca spp. (eg, Festuca rubra) and Poa spp. (eg, Poa pratense). The compounds may stunt grasses without significant phytotoxic effects and without deleteriously affecting the appearance (particularly the colour) of the grass; this makes such compounds attractive for use on ornamental lawns and on grass verges. They may also have an effect on flower head emergence in, for example,grasses. The compounds can also stunt weed species present in the grasses; examples of such

weed species are sedges (eg, Cyperus spp.) and dicotyledonous weeds (eg. daisy. plantain. knotweed. speedwell, thistle, docks and ragwort). The growth of non-crop vegetation (eg, weeds or cover vegetation) can be retarded thus assisting in the maintenance of plantation and field crops. In fruit orchards. particularly orchards subject to soil erosion, the presence of grass cover is important. However excessive grass growth requires substantial maintenance. The compounds of the invention could be useful in this situation as they could restrict growth without killing the plants which would lead to soil erosion: at the same time the degree of competition for nutrients and water by the grass would be reduced and this could result in an increased yield of fruit. In some cases, one grass species may be stunted more than another grass species: this selectivity could be useful, for example, for improving the quality of a sward by preferential suppression of the growth of undesirable species.

The dwarfing may also be useful in miniaturising ornamental. household, garden and nursery plants (eg. poinsettias, chrysanthemums, carnations, tulips and daffodils).

As indicated above, the compounds can also be used to stunt woody species. This property can be used to control hedgerows or to shape or reduce the need for pruning, of fruit trees (eg. apples. pears. cherries, peaches, vines etc).

The plant growth regulating effect may (as implied above) manifest itself in an increase in crop yield: or in an ability in orchards and other crops to increase fruit set, pod set and grain set.

Some coniferous trees are not significantly stunted by the compounds so the compounds could be useful in controlling undesirable vegetation in conifer nurseries.

In the potato, vine control in the field and inhibition of sprouting in the store may be possible.

In addition the compounds may be useful as abscission agents resulting in thinning of fruit on the tree and an increase in fruit quality.

Other plant growth regulating effects caused by the compounds include alteration of leaf angle and changes in leaf morphology (both of which may permit increased light interception and utilization) and promotion of tillering in monocotyledonous plants. Improved light interception is of value in all major world crops, eg, wheat, barley, rice, maize, soya, sugarbeet, potatoes. plantation crops and orchard crops. The leaf angle effect may be useful for example in altering the leaf orientation of. for example. potato crops thereby letting more light into the crops and inducing an increase in photosynthesis and tuber weight. By increasing tillering in monocotyledonous crops (eg, rice), the number of flowering shoots per unit area may be increased thereby increasing the overall grain yield of such crops. In addition better control and modification of hierarchical relationships is possible both in vegetative and reproductive stages of monocotyledonous and dicotyledenous plant growth, especially in cereals such as wheat, barley. rice and maize. whereby the number of flowering shoots per unit area may be increased and the size distribution of grains within the ear may be modified in such a way as to increase yield. In the treatment of rice plants. or rice crops the invention compounds can be applied, eg, as granules or a granular formulation. for example as slow release granules, to nursery boxes, paddy water and other like cultivation loci and media. In grass swards, especially amenity grass, an increase in tillering could lead to a denser sward which may result in increased resilience in wear; and to increased yields and better quality of forage grass, eg, improved digestability and palatability.

The treatment of plants with the compounds can lead to the leaves developing a darker green colour. In dicotyledonous plants such as soyabean and cotton, there may be promotion of sideshooting.

The compounds may inhibit, or at least delay, the flowering of sugar beet (and thereby may increase sugar yield) or otherwise modify the flowering patterns in many other crops. They may also reduce the size of sugar beet without reducing significantly the sugar yield thereby enabling an increase in planting density to be made. Similarly in other root crops (eg, turnip, swede, mangold, parsnip, beetroot. yam and cassava) it may be possible to increase the planting density.

The compounds could be useful in restricting the vegetative growth of cotton thereby leading to an increase in cotton yield. Crop yields may also be increased by improvement of the harvest index (ie. the harvested yield as a proportion of the total dry matter produced) by altering dry matter partitioning. This applies to all the aforementioned root, pod, cereal, tree. plantation and orchard crops.

The compounds may be useful in rendering plants resistant to stress since the compounds can delay the emergence of plants grown from seed, shorten stem height and delay flowering; these properties could be useful in preventing frost damage in countries where there is significant snow cover in the winter since then the treated plants would remain below snow cover during the cold weather. Further the compounds may cause drought or cold resistance in certain plants.

When applied as seed treatments at low rates the compounds can have a growth stimulating effect on plants.

It is to be understood that not all the compounds of the present invention will necessarily show all the

above mentioned plant growth regulating effects. Thus whilst there may be advantages in compounds which have a broad spectrum of plant growth regulating effects against a wide range of species. compounds having a high specific activity with respect to a particular species and or plant growth regulating effect may also be of great benefit.

In carrying out the plant growth regulating method of the invention. the amount of compound to be applied to regulate the growth of plants will depend upon a number of factors. for example the particular compound selected for use, and the identity of the plant species whose growth is to be regulated. However. in general an application rate of 0.1 to 15, preferably 0.1 to 5, kg per hectare is used. With the use of biodegradable polymeric slow release granules rates of 1 to 10g per hectare are feasible: whilst electrodynamic spraying techniques may also deploy lower rates of application. However. on certain plants even application rates within these ranges may give undesired phytotoxic effects. Routine tests may be necessary to determine the best rate of application of a specific compound for any specific purpose for which it is suitable.

The compounds may be used as such for plant growth regulating purposes but are more conveniently formulated into compositions for such usage. The invention thus provides a plant growth regulating composition comprising a compound of general formula (I) or (II) as hereinbefore defined. or a salt or metal complex thereof: and, optionally, a carrier or diluent.

The invention also provides a method of regulating plant growth. which comprises applying to the plant. to seed of a plant or to the locus of a plant or seed. a compound. or a salt or metal complex thereof. as hereinbefore defined. or a composition combining the same.

The compounds, salts, metal complexes, ethers and esters can be applied in a number of ways. for example they can be applied, formulated or unformulated. directly to the foliage of a plant. or they can be applied also to bushes and trees. to seeds or to other medium in which plants. bushes or trees are growing or are to be planted. or they can be sprayed on, dusted on or applied as a cream or paste formulation. or they can be applied as a vapour: or as slow release granules. Application can be to any part of the plant, bush or tree, for example to the foliage, stems, branches or roots. or to soil surrounding the roots. or to the seed before it is planted; or to the soil generally, to paddy water or to hydroponic culture systems. The invention compounds may also be injected into plants or trees and they may also be sprayed onto vegetation using electrodynamic spraying techniques.

The term "plant" as used herein includes seedlings, bushes and trees.

The compounds are preferably used for agricultural and horticultural purposes in the form of a composition. The type of composition used in any instance will depend upon the particular purpose envisaged.

The compositions may be in the form of dusting powders or granules comprising the active ingredient and a solid diluent or carrier, for example fillers such as kaolin, bentonite, kieselguhr, dolomite, calcium carbonate, talc, powdered magnesia, Fuller's earth, gypsum, Hewitt's earth, diatomaceous earth and China clay. Such granules can be preformed granules suitable for application to the soil without further treatement. These granules can be made either by impregnating pellets of filler with the active ingredient or by pelleting a mixture of the active ingredient and powdered filler. Compositions for dressing seed, for example. may comprise an agent (for example a mineral oil) for assisting the adhesion of the composition to the seed: alternatively the active ingredient can be formulated for seed dressing purposes using an organic solvent (for example N-methylpyrrolidone or dimethylformamide).

The compositions of the present invention may also be in the form of dispersible powders, granules or grains comprising a wetting agent to facilitate the dispersion in liquids of the powder or grains which may contain also fillers and suspending agents.

The aqueous dispersions or emulsions may be prepared by dissolving the active ingredient(s) in an organic solvent optionally containing wetting, dispersing or emulsifying agent(s) and then adding the mixture to water which may also contain wetting, dispersing or emulsifying agent(s). Suitable organic solvents are ethylene dichloride, isopropyl alcohol, propylene glycol, diacetone alcohol, toluene, kerosene, methylnaphthalene, the xylenes, trichloroethylene, furfuryl alcohol, tetrahydrofurfuryl alcohol, and glycol ethers (eg, 2-ethoxyethanol and 2-butoxyethanol).

The compositions to be used as sprays may also be in the form of aerosols wherein the formulation is held in a container under pressure in the presence of a propellant, eg, fluorotrichloromethane or dichlorodifluoromethane.

The compounds can be mixed in the dry state with a pyrotechnic mixture to form a composition suitable for generating in enclosed spaces a smoke containing the compounds.

Alternatively, the compounds may be used in a micro-encapsulated form. They may also be formulated in biodegradable polymeric formulations to obtain a slow, controlled release of the active substance.

By including suitable additives, for example additives for improving the distribution. adhesive power and resistance to rain on treated surfaces. the different compositions can be better adapted for various utilities.

The compounds can be used as mixtures with fertilisers (eg. nitrogen-. potassium-or phosphorus-containing fertilisers). Compositions comprising only granules of fertiliser incorporating, for example coated with, the compound are preferred. Such granules suitably contain up to 25% by weight of the compound. The invention therefore also provides a fertiliser composition comprising the compound of general formula (I) or (II) or a salt or metal complex thereof.

The compositions may also be in the form of liquid preparations for use as dips or sprays which are generally aqueous dispersions or emulsions containing the active ingredient in the presence of one or more surfactants eg, wetting agent(s), dispersing agent(s), emulsifying agent(s) or suspending agent(s): or which are spray formulations of the kind suitable for use in electrodynamic spraying techniques. The foregoing agents can be cationic, anionic or non-ionic agents. Suitable cationic agents are quaternary ammonium compounds, for example cetyltrimethylammonium bromide.

Suitable anionic agents are soaps, salts of aliphatic monoesters of sulphuric acid (for example sodium lauryl sulphate), and salts of sulphonated aromatic compounds (for example sodium dodecylbenzenesulphonate, sodium, calcium or ammonium lignosulphonate. butylnaphthalene sulphonate. and a mixture of sodium diisopropyl-and triisopropyl-naphthalene sulphonates).

Suitable non-ionic agents are the condensation products of ethylene oxide with fatty alcohols such as oleyl or cetyl alcohol, or with alkyl phenols such as octyl-or nonyl-phenol and octylcresol. Other non-ionic agents are the partial esters derived from long chain fatty acids and hexitol anhydrides. the condensation products of the said partial esters with ethylene oxide, and the lecithins. Suitable suspending agents are hydrophilic colloids (for example polyvinylpyrrolidone and sodium carboxymethylcellulose), and the vegetable gums (for example gum acacia and gum tragacanth).

The compositions for use as aqueous dispersions or emulsions are generally supplied in the form of a concentrate containing a high proportion of the active ingredient(s), and the concentrate is to be diluted with water before use. These concentrates often should be able to withstand storage for prolonged periods and after such storage be capable of dilution with water in order to form aqueous preparations which remain homogeneous for a sufficient time to enable them to be applied by conventional and electrodynamic spray equipment. The concentrates may conveniently contain up to 95%. suitably 10-85%. for example 25-60%. by weight of the active ingredient(s). These concentrates suitably contain organic acids (eg. alkaryl or aryl sulphonic acids such as xylenesulohonic acid or dodecyl benzenesulphonic acid) since the presence of such acids can increase the solubility of the active ingredient(s) in the polar solvents often used in the concentrates. The concentrates suitably contain also a high proportion of surfactants so that sufficiently stable emulsions in water can be obtained. After dilution to form aqueous preparations. such preparations may contain varying amounts of the active ingredient(s) depending upon the intended purpose, but an aqueous preparation containing 0.0005% to 10%. or 0.01% to 10%. by weight of active ingredient(s) may be used.

The compositions of this invention can comprise also one or more additional compound(s) having biological activity, eg, compounds having similar or complementary fungicidal or plant growth activity or compounds having plant growth regulating, herbicidal or insecticidal activity.

The additional fungicidal compound can be, for example, one which is capable of combating ear diseases of cereals (eg,wheat) such as Septoria, Gibberella and Helminthosporium spp.. seed and soil borne diseases and downy and powdery mildews on grapes and powdery mildew and scab on apple etc. Examples of suitable additional fungicidal compound are imazalil, benomyl. carbendazim, thiophanate-methyl, captafol, captan, sulphur, triforine. dodemorph. tridemorph. pyrazophos, furalaxyl. ethirimol. tecnazene, dimethirimol, bupirimate, chlorothalonil, vinclozolin, procymidone. iprodione, metalaxyl. forsetyl aluminium, carboxin, oxycarboxin, fenarimol, nuarimol, fenfuram, methfuroxan, nitrotal-isopropyl. triadimefon. thiabendazole. etridiazole. triadimenol, biloxazol, dithianon. binapacryl, quinomethionate. guazatine, dodine fentin acetate, fentin hydroxide, dinocap, folpet, dichlofluanid, ditalimphos, kitazin. cycloheximide. dichlobutrazol, a dithiocarbamate, a copper compound. a mercury compound, 1-(2-cyano-2-methoxyiminoacetyl)-3-ethyl urea, fenaponil, ofurace, propiconazole, etaconazole and fenpropemorph and fenpropidine.

The compounds of general formula (I) or (II) can be mixed with soil, peat or other rooting media for the protection of plants against seed-borne, soil-borne or foliar fungal diseases.

Suitable additional insecticides are Pirimor, Croneton, dimethoate, Metasystox. pyrethroid insecticides and formothion.

The other, additional, plant growth regulating compound can be one which controls weeds or seedhead formation, improves the level or longevity of the plant growth regulating activity of the compounds of

general formula (I) or (II), selectively controls the growth of the less desirable plants (eg. grasses) or causes the compound of general formula (I) or (II) to act faster or slower as a plant growth regulating agent. Some of these other agents will alsobe herbicides.

Examples of suitable plant growth regulating compounds, which can display synergy in admixture, or use, with the invention compounds are the gibberellins (eg. $GA_3$, $GA_4$ or $GA_7$), the auxins (eg. indoleacetic acid, indolebutyric acid, naphthoxyacetic acid or naphthylacetic acid), the cytokinins (eg. kinetin, diphenylurea, benzimidazole, benzyladenine or benzylaminopurine), phenoxyacetic acids (eg. 2,4-D or MCPA), substituted benzoic acids (eg. triiodobenzoic acid), morphactins (eg. chlorfluorecol), maleic hydrazide, glyphosate, glyphosine, long chain fatty alcohols and acids, dikegulac, fluoridamid, mefluidide, substituted quaternary ammonium and phosphonium compounds (eg. chlormequat* chlorphonium or mepiquat chloride*), ethephon, carbetamide, methyl-3,6-dichloroanisate, daminozide*, asulam, abscisic acid, isopyrimol, 1-(4-chlorophenyl)-4,6-dimethyl-2-oxo-1,2-dihydropyridine-3-carboxylic acid, hydroxybenzonitriles (eg, bromoxynil), difenzoquat*, benzoylprop-ethyl 3,6-dichloropicolinic acid, fenpentezol, triapenthanol, flurpirimidol, paclobutrazol, tetcyclacis and tecnazene. Synergy will be most likely to occur with those of the foregoing which are quaternary ammonium compounds and with those marked with an asterisk.

For certain applications, for example in the injection of the compounds of the invention into trees or plants, it is desirable that the compounds have a relatively high solubility in water, for example a solubility in excess of 30 parts per million. The compounds may alternatively be injected into the tree in the form of an organic solution, for example a solution in a lower alcohol.

For certain applications it is also desirable that the compound has a low persistancy in soil to prevent carry-over to adjacent crops or even crops planted subsequently in the same soil. Preferably the compound for use in such applications has a half life in the soil of less than 20 weeks.

The invention is illustrated by the following Examples, in which infra red characterisation of the compounds is given as λ max ($cm^{-1}$); NMR characterisation of the compounds is given in terms of $\delta_H$; and mass spectroscopy analysis is given in terms of m/z.

EXAMPLE 1

This Example illustrates the preparation of 1-(1,2,4-triazol-1-yl)-2-hydroxy-2-(tertiary butyl)-2-(5-propyl thiophen-2-yl)-ethane (Compound No. 1 of Table I).

To a solution of 2-propylthiophene (1 equivalent, 1.93g, 15.3mmol) in dry tetrahydrofuran (2.5ml per mmol) at 0°C and in an inert atmosphere was added n-butyl lithium (1 equivalent). After 15 minutes the solution was cooled to -78°C and chlorotitanium tri-isopropoxide (1 equivalent) was added. Then a solution of triazolyl pinacolone (1.1 equivalent, 1.64g, 16.8mmol) in dry tetrahydrofuran (3ml per mmol) was added and the reaction mixture allowed to slowly warm to room temperature (16 hours). The mixture was poured into water and extracted with ether (2 × 250ml). The organic layer was separated, dried over magnesium sulphate, and the solvent removed. Flash chromatography (silica gel, petrol/ethyl acetate elution) of the residue gave the desired product as a pale yellow gum (1.0g, 22.4%).

Analysis: C, 61.2; H, 8.0; N, 14.2.

$C_{15}H_{23}N_3OS$ requires C, 61.4; H, 7.9; N, 14.3%.

Infra red 3400, 2900, 1450, 1370, 1265, 1190, 1140, 1010, 955, 850, 800, 725, 680, 640, 610 $cm^{-1}$;

NMR (90 MHz; $CDCl_3$) 0.91 (3H, t, J=6.3 Hz), 1.12 (9H, s), 1.6 (2H, sx, J=6.3 Hz), 2.66 (2H, t, J=6.3 Hz), 4.0 (1H, brs), 4.6 (2H, dd, J=14.4 and 16.2 Hz), 6.55 (1H, m), 6.64 (1H, d, J=3.6 Hz), 7.83 (1H, s), 7.86 (1H, s);

m/z 293 ($M^+$), 250, 236 (100%), 211.

EXAMPLE 2

This Example illustrates the preparation of 1-(1,2,4-triazol-1-yl)-2-hydroxy-2-(tertiary butyl)-2-(5-methylthiophen-2-yl)-ethane (Compound No. 2 of Table I).

Using the general method of Example 1, 2-methylthiophene (1.0g, 10.2mmol) and triazolyl pinacolone (1.76g, 11.2mmol) were reacted to give the product as a pale yellow solid (400mg, 15%), melting point 97-100°C.

Analysis Found: C, 58.6; H, 7.8; N, 15.3.

$C_{13}H_{19}N_3OS$ requires C, 58.8; H, 7.2; N, 15.8%;

Infra red 3400, 2900, 1450, 1370, 1265, 1050, 950, 805, 700, 680, 655, 600 $cm^{-1}$;
NMR (90 MHz; $CDCl_3$) 1.10 (9H, s), 2.34 (3H, d, J = 1.8 Hz), 4.58 (2H, d, J = 2.7 Hz), 4.70 (1H, s), 6.52 (1H, m), 6.58 (1H, d, J = 3.6 Hz), 7.84 (1H, s), 7.86 (1H, s);
m/z 265 ($M^+$), 208 (100%), 183.

EXAMPLE 3

This Example illustrates the preparation of 1-(1,2,4-triazol-1-yl)-2-hydroxy-2-(tertiary butyl)-2-(5-ethylthiophen-2-yl)-ethane (Compound No. 3 of Table I).

Using the general method of Example 1, 2-ethylthiophene (1.71g, 15.3mmol) and triazolyl pinacolone (2.64g, 16.8mmol) were reacted to give the product as a pale yellow solid (800mg, 18.7%), melting point 67-69°C.

Analysis Found: C, 59.8; H, 8.0; N, 14.4.
$C_{14}H_{21}N_3OS$ requires C, 60.2; H, 7.6; N, 15.0%;
Infra red 3400, 2900, 1450, 1370, 1265, 1190, 1140, 1005, 950, 810, 710, 680, 655, 600 $cm^{-1}$;
NMR (90 MHz; $CDCl_3$) 1.10 (9H, s), 1.2 (3H, t, J = 6.5 Hz), 2.70 (2H, t, J = 6.5 Hz), 4.56 (2H, dd, J = 14.4 and 16.2 Hz), 4.68 (1H, s), 6.52 (1H, m), 6.58 (1H, d, J = 3.6 Hz), 7.82 (1H, s), 7.84 (1H, s);
m/z 280 ($MH^+$), 222 (100%), 197.

EXAMPLE 4

This Example illustrates the preparation of 1-(1,2,4-triazol-1-yl)-2-hydroxy-2-(tertiary butyl)-4-(thiophen-2-yl)-but-3-yne (Compound No. 4 of Table I).

To a solution of 1-(thiophen-2-yl)-2,2-dibromoethylene (prepared as described in Liebigs Ann.Chem. 1980, 2061) (2.0g, 746mmol) in dry tetrahydrofuran (25ml) at -78°C was added n-butyl lithium (5.97ml of 2.5M, 14.9mmol) under an inert atmosphere. After 35 minutes the solution was allowed to warm to room temperature. After a further 35 minutes the solution was cooled to -78°C and chlorotitanium tri-iso-propoxide (7.46ml of 1M, 7.46mmol) was added. After 15 minutes a solution of triazolyl pinacolone (1.5g, 9mmol) in dry tetrahydrofuran was added and the solution allowed to warm slowly to room temperature (16 hours). The reaction mixture was then poured into water and extracted with dichloromethane (2 × 250ml). The organic layer was collected, dried over magnesium sulphate and the solvent removed. Flash chromatography (silica gel, petrol/ether acetate elution) of the resulting residue gave the desired product (0.35g, 17%), melting point 110-114°C.

Analysis Found: C, 62.2; H, 6.5; N, 13.5.
$C_{14}H_{17}N_3OS$ requires C, 61.1; H, 6.2; N, 15.3%;
NMR (90 MHz; $CDCl_3$) 1.18 (9H, s), 4.10 (1H, s), 4.42 (2H, s), 6.82 (1H, dd, J = 4.5 and 3.6 Hz), 7.16 (1H, dd, J = 3.6 and 1.8 Hz), 7.22 (1H, dd, J = 4.5 and 1.8 Hz), 8.01 (1H, s), 8.26 (1H, s);
m/z 275 ($M^+$), 274, 218 (100%), 82.

EXAMPLE 5

This Example illustrates the preparation of 1-(1,2,4-triazol-1-yl)-2-hydroxy-2-(tertiary butyl)-3-(5-methylthiophen-2-yl)-propane (Compound No. 5 of Table I).

To a solution of 2-methylthiophene (3 equivalents - 1.08g, 11.0mmol) in dry ether (0.66ml per mmol) at 0°C and under an inert atmosphere was added n-butyl lithium (3 equivalents). After 25 minutes a solution of 3,3-dimethyl-2-(1,2,4-triazol-1-yl-methyl)-1,2-epoxy-butane (1 equivalent, 1.0g, 5.0mmol) in dry ether (1ml per mmol) was added and the reaction stirred for 16 hours. The reaction mixture was then poured into water and extracted with ether (2 × 250ml). The organic layer was collected, dried over magnesium sulphate and the solvent removed. Flash chromatography (silica gel, petrol/ether elution) gave the desired product as a pale yellow solid (0.27g, 19.4%), melting point 91-94°C.

Analysis Found: C, 60.9; H, 8.1; N, 15.1.
$C_{14}H_{21}N_3OS$ requires C, 60.2; H, 7.6; N, 15.0%;

Infra red 3250. 2900, 1450. 1370. 1260. 1125. 960. 870. 805. 715. 600 $cm^{-1}$ :
NMR (90 MHz; $CDCl_3$) 0.98 (9H. s), 2.4 (3H, s), 3.07 (2H. s). 3.32 (1H. s), 4.30 (2H. s). 6.51 (2H. m). 7.94 (2H. broad s);
m/z 280 ($MH^+$). 279. 222. 168 (100%), 111, 70, 57, 43.

EXAMPLE 6

1-(1.2,4-Triazol-1-yl)-2-hydroxy-2-(tertiary butyl)-3-(5-ethyl-thiophen-2-yl)-propane (Compound No. 6 of Table I) was prepared using the general method of Example 5. The product was a pale yellow solid (0.85g. 58%), melting point 75-77°C.
Analysis Found: C, 61.6; H, 8.1; N, 14.5.
$C_{15}H_{23}N_3OS$ requires C, 61.4; H, 7.9; N, 14.3%;
Infra red 3200, 2900, 1450, 1370, 1270, 1200, 1130, 1090, 1010. 960, 880. 820. 720, 680. 600 $cm^{-1}$ :
NMR (90 MHz; $CDCl_3$) 1.0 (9H, s), 1.26 (3H, t, J = 7.2 Hz), 2.76 (2H, q, J = 7.2 Hz), 3.09 (2H, s), 3.35 (1H, s), 4.32 (2H, s), 6.51 (1H, d, J = 3.6 Hz), 6.59 (1H, d, J = 3.6 Hz), 7.88 (1H, s), 7.92 (1H, s);
m/z 293 ($M^+$), 168 (100%).

EXAMPLE 7

1-(1.2,4-Triazol-1-yl)-2-hydroxy-2-(tertiary butyl)-3-(5-propyl-thiophen-2-yl)-propane (Compound No. 7 of Table I) was prepared using the general method of Example 5. The product was a pale yellow solid (0.69g, 50%), melting point 46-48°C.
Analysis Found: C, 62.9; H, 8.6; N, 14.2.
$C_{16}H_{25}N_3OS$ requires C, 62.5; H, 8.2; N, 13.7%;
Infra red 3200, 2900, 1450, 1370, 1265, 1200, 1120, 1090, 1005, 960, 880, 850, 805, 780, 670, 640, 600 $cm^{-1}$ ;
NMR (90 MHz: $CDCl_3$) 0.94 (3H, t, J = 7.3 Hz), 1.0 (9H, s), 1.64 (2H, sx, J = 7.3 Hz), 2.68 (2H, t, J = 7.3 Hz), 3.07 (2H, d, J = 2.7 Hz), 3.32 (1H, s), 4.30 (2H, s), 6.50 (1H, d, J = 3.6 Hz), 6.58 (1H, d, J = 3.6 Hz), 7.90 (2H, br);
m/z 308 ($MH^+$), 307, 168 (100%).

EXAMPLE 8

This Example illustrates the preparation of 1-(1,2,4-triazol-1-yl)-2-hydroxy-2-(tertiary butyl)-4-(thiophen-2-yl)-butane (Compound No. 8 of Table I).

Stage 1 : Preparation of 4.4-dimethyl-1-(thien-2-yl)pent-1-en-3-one

Using the method disclosed in Chem.Abs. 1972. 77 87679, 2-thiophenaldehyde (5g, 44.6mmol) and pinacolone (10ml, 80mmol) were added to a solution of sodium hydroxide (2g, 50mmol) in ethanol/water (50/100ml) and stirred for 24 hours. The mixture was then poured into water and extracted with ether (2 × 250ml). The organic layer was collected, dried over magnesium sulphate and the solvent removed. This gave the product as a yellow oil (8.0g, 92.5%).

Stage 2 : Preparation of 4,4-dimethyl-1-(thien-2-yl)-pentan-3-one

To a solution of the product of Stage 1 (4g, 20.6mmol) in ethanol (100ml) was added palladium/charcoal (1g of 10%) and the reaction vessel charged with hydrogen (100psi). After 3 days the catalyst was filtered off and the solvent removed. Flash chromatography (silica gel, petrol/dichloromethane elution) gave the product (2.8g, 70%).

Stage 3

To a solution of the product of Stage 2 (2g, 10.2mmol) in toluene (12ml) was added trimethylsulphoxonium iodide (2.9g, 13.2mmol), a catalytic amount of tetrabutylammonium bromide (50mg) and potassium hydroxide solution (5ml of 50%). The reaction was heated to 80°C for 4 hours. On cooling, the reaction was poured into water and extracted wth ether (2 × 250ml). The organic layer was collected, dried over magnesium sulphate, and the solvent removed. The resulting oil was used without further purification and was added to a mixture of 1,2,4-triazole (0.7g, 10.2mmol) and potassium carbonate (1.4g, 10.2mmol) in dry dimethylformamide (15ml). The reaction was heated to 80°C for 16 hours. On cooling, the mixture was poured into water and extracted with ether (2 × 250ml). The organic phase was collected, dried over magnesium sulphate, and the solvent removed. Flash chromatography (silica gel, petrol ether elution) gave the product (2.1g, 74%).

Analysis Found: C, 60.1; H, 8.1; N, 15.1.

$C_{14}H_{21}N_3OS$ requires C, 60.2; H, 7.6; N, 15.0%;

Infra red 3330, 2950, 1450, 1370, 1270, 1200, 1130, 1080, 1020, 955, 870, 845, 815, 690, 645, 600 $cm^{-1}$;

NMR (90 MHz; $CDCl_3$) 1.0 (9H, s), 1.7-2.2 (3H, m), 2.5-2.85 (1H, m), 3.14 (1H, s), 4.31 (2H, s), 6.34 (1H, dd, J=3.6 and 1.8 Hz), 6.86 (1H, dd, J=4.5 and 3.6 Hz), 7.06 (1H, dd, J=4.5 and 1.8 Hz), 7.97 (1H, s), 8.18 (1H, s);

m/z 279 ($M^+$), 261, 222, 197, 97 (100%).

EXAMPLE 9

1-(1,2,4-Triazol-1-yl)-2-hydroxy-2-(tertiarybutyl)-4-(thiophen-3-yl)butane (Compound No. 9 of Table I) was prepared using a method corresponding to that of Example 8, except that 3-thiophenaldehyde was used as starting material instead of 2-thiophenaldehyde. The product (1.4g, 49%) was characterised as follows :

Analysis Found:C, 60.2; H, 8.3; N, 14.9.

$C_{14}H_{21}N_3OS$ requires C, 60.2; H, 7.6; N, 15.0%;

Infra red 3350, 2900, 1450, 1370, 1280, 1260, 1190, 1135, 1075, 1020, 955, 900, 855, 820, 800, 760, 720, 680, 620, 600 $cm^{-1}$.

NMR (90 MHz; $CDCl_3$) 1.01 (9H, s), 1.16-2.05 (3H, m), 2.4-2.7 (1H, m), 3.02 (1H, s), 4.35 (2H, s), 6.82 (1H, d, J=3.6 Hz), 6.84 (1H, s), 7.19 (1H, d, J=3.6 Hz), 7.99 (1H, s), 8.19 (1H, s);

m/z 279 ($M^+$), 222, 192, 97 (100%).

EXAMPLE 10

This Example illustrates the preparation of 1-(1,2,4-triazol-1-yl)-2-hydroxy-2-(tertiarybutyl)-3-(5-methylfuran-2-yl)ethane (Compound No. 10 of Table I).

A mixture of 2-methylfuran (1.39g, 16.5mmol), trimethylacetic anhydride (3.27g, 20mmol) and boron trifluoride etherate (1 ml) was heated at 60°C for 3 hours. On cooling to room temperature, water (50 ml) was added and stirred for 2 hours. The mixture was extracted with chloroform (150 ml), the organic fraction was washed with potassium carbonate solution, collected and dried over magnesium sulphate. The solvent was removed to give an oil which was dissolved in dry DMSO (30 ml). To this was added trimethylsulphonium iodide (4.03g, 19.8mmol) and powdered potassium hydroxide (1.9g, 33mmol) and stirred for 16 hours. The reaction was then poured into water, extracted with ether (2 × 100 ml), washed with water (150 ml), the organic fraction collected and dried over magnesium sulphate. On removing the solvent the resulting oil was dissolved in dry DMF (20 ml) and to this added 1,2,4-triazole (1.13g, 16.4mmol) and potassium carbonate (2.26g, 16.4mmol). The mixture was heated to 80°C and stirred for 16 hours. The reaction was then poured into water, extracted with ether (2 × 100 ml), washed with water (150 ml), the organic fraction was collected and dried over magnesium sulphate. On removing the solvent the resulting oil was purified by flash chromatography (silica gel, petrol ether elution) to give the product as a white solid (0.33g, 8%).

Analysis C, 62.9; H, 7.0; N, 14.8.

$C_{13}H_{19}N_3O$ requires C, 62.6; H, 7.0; N, 16.9%.

Infra red 3250, 2950, 1280, 1200, 1130, 750, 660 $cm^{-1}$.
NMR (90 MHz; $CDCl_3$) 1.0 (9H, s), 2.36 (3H, s), 2.8 (1H, s), 4.3 (1H, d), 4.66 (1H, d, J=11.7 Hz), 6.0 (1H, d, J=2.7 Hz), 6.3 (1H, d, J=2.7 Hz), 7.95 (1H, s), 8.48 (1H, s);
m/z 249 (M ), 192, 175 (100%), 162, 123, 95.

EXAMPLE 11

This Example illustrates the preparation of 1-(1,2,4-triazol-1-yl)-2-hydroxy-2-(tertiarybutyl)-3-(5-ethyl-furan-2-yl)ethane (Compound No. 11 of Table I) and 1-(1,3,4-triazol-1-yl)-2-hydroxy-2-(tertiarybutyl)-3-(5-ethyl-furan-2-yl)ethane (Compound No. 12 of Table I).

A mixture of 2-ethylfuran (1.59g, 16.5mmol), trimethylacetic anhydride (3.27g, 20mmol) and boron trifluoride ethereate (1 ml) was heated at 60°C for 3 hours. On cooling to room temperature water (50 ml) was added and stirred for 2 hours. The mixture was extracted with chloroform (150 ml), the organic fraction was washed with potassium carbonate solution, collected and dried over magnesium sulphate. The solvent was removed to give an oil which dissolved in dry DMSO (30 ml). To this was added trimethylsulphonium iodide (4.03g, 19.8mmol) and powdered potassium hydroxide (1.9g, 33mmol) and the mixture was stirred for 16 hours. The reaction was then poured into water, extracted with ether (2 × 100 ml), washed with water (150 ml), the organic fraction was collected and dried over magnesium sulphate. On removing the solvent the resulting oil was dissolved in dry DMF (20 ml) and to this was added 1,2,4-triazole (1.13g, 16.4 mmol) and potassium carbonate (2.26g, 16.4 mmol). The mixture was heated to 80°C and stirred for 16 hours. The reaction was then poured into water and extracted with ether (2 × 100 ml), washed with water (150 ml) and, the organic fraction was collected and dried over magnesium sulphate. On removing the solvent the resulting oil was purified by flash chromatography (silica gel, petrol ether elution) to give both compounds 11 and 12 as white solids (compound 11, 0.27g, 6.2%; compound 12, 0.2g, 4.6%).

Compound 11 Analysis C, 63.2; H, 8.5; N, 15.7.
$C_{14}H_{21}N_3O_2$ requires C, 63.85; H, 8.0; N, 16.0%.
Infra red 3200, 2900, 1450, 1370, 1275, 1200, 1135, 1075, 960, 800, 760, 660 $cm^{-1}$;
NMR (90 MHz; $CDCl_3$) 1.0 (9H, s), 1.27 (3H, d, J=5.6 Hz), 2.6 (1H, s), 2.69 (2H, q, J=5.6 Hz), 4.28 (1H, d, J=11.7 Hz), 4.68 (1H, d, J=11.7 Hz), 6.0 (1H, d, J=2.7 Hz), 6.3 (1H, d, J=2.7 Hz), 7.95 (1H, s), 8.45 (1H, s);
m/z 263 (M ), 206, 189 (100%), 176, 137, 57.

Compound 12 Analysis C, 62.2; H, 7.9; N, 14.0.
$C_{14}H_{21}N_3O_2$ requires C, 63.85; H, 8.0; N, 16.0%.
Infra red 3150, 2900, 1450, 1370, 1270, 1200, 1105, 1005, 790, 730, 600 $cm^{-1}$;
NMR (90 MHz; $CDCl_3$) 1.06 (9H, s), 1.19 (3H, d, J=5.6 Hz), 1.8 (1H, s), 2.55 (2H, q, J=5.6 Hz), 4.38 (1H, d, J=11.7 Hz), 4.88 (1H, d, J=11.7 Hz), 5.75 (1H, d, J=2.7 Hz), 6.0 (1H, d, J=2.7 Hz), 7.76 (1H, s), 7.8 (1H, s);
m/z 263 (M ), 206 (100%), 181, 109, 82.

EXAMPLE 12

This Example illustrates the preparation of 1-(1,2,4-triazol-1-yl)-2-hydroxy-2-(tertiarybutyl)-4-(tetrahydrofuran-2-yl)butane (Compound No. 13 of Table I).

Stage 1 : Preparation of 4,4-dimethyl-1-(2-furyl)pent-1-en-3-one

2-Furaldehyde (4.3g, 44.6 mmol) and pinacolone (10 ml, 80mmol) were added to a solution of sodium hydroxide (2g, 50mmol) in ethanol:water (50:100 ml) and stirred for 24 hours. The mixture was then poured into water and extracted with ether (2 × 250 ml). The organic layer was collected, dried over magnesium sulphate and the solvent removed. This gave the product as a yellow oil.

Stage 2 : Preparation of 4,4-dimethyl-1-(2-tetrahydrofuryl)pentan-3-one

To a solution of 4,4-dimethyl-1-(2-furyl)pent-1-en-3-one (4g. 22.5mmol) in ethanol (100 ml) was added palladium charcoal (1g of 10%) and the reaction vessel charged with hydrogen (100 psi). After 24 hours the catalyst was screened off and the solvent removed. Flash chromatography (silica gel. Merck Art. 7736. petrol/dichloromethane elution) gave the fully saturated product (1.5g, 35%).

Stage 3 : Preparation of 1-(1,2,4-triazol-1-yl)-2-hydroxy-2-(tertiarybutyl)-4-(tetrahydrofuran-2-yl)butane

To a solution of 4,4-dimethyl-1-(2-tetrahydrofuryl)-pentan-3-one (1.5g, 8mmol) in DMSO (20 ml) was added trimethylsulphonium iodide (2.01g, 9.8mmol) and potassium hydroxide (1g, 17.8mmol). The reaction was stirred at room temperature for 24 hours. The reaction was poured into water and extracted with ether (2 × 250 ml). The organic fraction was collected, dried over magnesium sulphate, and the solvent removed. The resulting oil was used without further purification. To a mixture of 1,2,4-triazole (0.7g, 10.2mmol) and potassium carbonate (1.4g, 10.2mmol) in dry DMF (15 ml) was added the oil. The reaction was heated to 80°C for 16 hours. On cooling, the mixture was poured into water and extracted with ether (2 × 250 ml). The organic phase was collected, dried over magnesium sulphate, and the solvent removed. Flash chromatography (silica gel, Merck Art. 7736, petrol/ether elution) gave the product (0.25g, 11.7%).

Analysis C, 61.7; H, 9.1; N, 14.8.

$C_{15}H_{25}N_3O_2$ requires C, 61.7; H. 9.4; N, 15.7%.

Infra red 3350, 2950, 1500, 1450, 1360, 1270, 1200, 1130, 1040, 955, 860, 675, 645, 600 $cm^{-1}$.

NMR (90 MHz; $CDCl_3$) 1.0 (9H, s), 0.6-1.2 (2H, m), 1.6-1.98 (6H, m), 3.5-3.84 (3H, m), 4.28 (2H, s), 4.41 (1H, s), 7.92 (1H, s), 8.25 (1H, s);

m/z 268 (M +H), 252, 210 (100%), 185, 168.

EXAMPLE 13

Compounds 1 and 4 to 9 in Table I were tested for plant growth regulator activity against five species for various growth effects relevant to plant growth regulation.

Methodology

The plant species used in this screen are presented in Table II with the leaf stage at which they were sprayed. Each chemical was applied at 4000 ppm (4 kg/ha in a 1000 l/ha field volume) using a tracksprayer and a SS8004E (Teejet) nozzle.

After spray, the plants were grown in a glasshouse with 25°C day/22°C night temperatures and supplementary lighting was supplied when necessary (from mercury vapour lamps), to provide a 16 hour photoperiod. The exception to this were the temperate cereals, wheat and barley which were grown in 16°C day/13°C night temperatures.

After 2-6 weeks in the glasshouse, depending on the time of year, the plants were visually assessed for morphological characteristics. Formulation blanks were used as controls to assess the plants. The results are presented in Table III.

## TABLE II

PLANT MATERIAL USED FOR WHOLE PLANT SCREEN

| SPECIES | CODE | VARIETY | GROWTH STAGE AT TREATMENT | NO. PLANTS PER 3" POT | COMPOST TYPE |
|---|---|---|---|---|---|
| Barley | BR | Atem | 1 - 1.5 leaves | 4 | JIP* |
| Wheat | WW | Timmo | 1 - 1.5 leaves | 4 | JIP |
| Maize | MZ | Earliking | $2\frac{1}{3}$ leaves | 1 | PEAT |
| Apple | AP | Red Delicious | 4 - 5 leaves | 1 | JIP |
| Rice | RC | Ishikari | 2 - $2\frac{1}{2}$ leaves | 4 | JIP |

JIP* = John Innes Potting Compost.

TABLE III

| SPECIES | COMPOUND NO. | R | G | A | T | I |
|---|---|---|---|---|---|---|
| WW | 1 | 2 | 1 | | | 2 |
| | 4 | 2 | | | 1 | 2 |
| | 5 | 2 | 1 | | 2 | 2 |
| | 6 | 2 | | | 2 | 3 |
| | 7 | | | | | |
| | 8 | 2 | | | 2 | 3 |
| | 9 | 2 | 1 | | 1 | 2 |
| BR | 1 | 1 | | | 1 | 1 |
| | 4 | | | | | |
| | 5 | 1 | | | | 1 |
| | 6 | | | | 1 | |
| | 7 | | | | | |
| | 8 | 1 | 1 | | | 1 |
| | 9 | 1 | | | | 1 |
| RC | 1 | 2 | 1 | | 1 | 3 |
| | 4 | 1 | | | | 2 |
| | 5 | 2 | 1 | | | 2 |

TABLE III (CONT/D)

| SPECIES | COMPOUND NO. | R | G | A | T | I |
|---|---|---|---|---|---|---|
| RC (continued) | 6 | 2 | 1 | | 1 | 2 |
| | 7 | | | | | |
| | 8 | 1 | | | | 2 |
| | 9 | 2 | | | | 3 |
| AP | 1 | | | | 1 | |
| | 4 | | | | | |
| | 5 | 2 | | 2 | 2 | |
| | 6 | 3 | 1 | | | 2 |
| | 7 | 3 | 1 | | 2 | 2 |
| | 8 | 3 | 1 | | | 2 |
| | 9 | 1 | 1 | | 2 | 1 |
| MZ | 1 | | | | | |
| | 4 | | | 2 | | |
| | 5 | | | 1 | | |
| | 6 | | | | | |
| | 7 | | 1 | 1 | | |
| | 8 | | | | | |
| | 9 | | | | | |

Key:

R = Retardation
G = Greening effect
A = Apical damage
T = Tillering or side shooting
I = Interligular or internodal length reduction All effects are scored visually on a 1-3 basis where

1 = 10-30%
2 = 31-60%
3 = 61-100% Blank means less than 10% effect.

EXAMPLE 14

Compounds 2 and 10 to 13 in Table I were tested for plant growth regulator activity against six species for various growth effects relevant to plant growth regulation.

Methodology

The plant species used in this screen are presented in Table IV with the leaf stage at which they were sprayed. Each chemical was applied at 4000 ppm (4 kg/ha in a 1000 1/ha field volume) using a tracksprayer and a SS8004E (Teejet) nozzle.

After spray, the plants were grown in a glasshouse with 25°C day/22°C night temperatures and supplementary lighting was supplied when necessary (from mercury vapour lamps), to provide a 16 hour photoperiod. The exception to this were the temperate cereals, wheat and barley which were grown in 16°C day/13°C night temperatures.

After 2-6 weeks in glasshouse, depending on the time of year, the plants were visually assessed for morphological characteristics. Formulation blanks were used as controls to assess the plants. The results are presented in Table V.

TABLE IV

PLANT MATERIAL USED FOR WHOLE PLANT SCREEN

| SPECIES | CODE | VARIETY | GROWTH STAGE AT TREATMENT | NO. PLANTS PER 3" POT | COMPOST TYPE |
|---|---|---|---|---|---|
| Barley | BR | Atem | 1 - 1.5 leaves | 4 | JIP* |
| Wheat | WW | Timmo | 1 - 1.5 leaves | 4 | JIP |
| Maize | MZ | Earliking | 2¼ leaves | 1 | PEAT |
| Apple | AP | Red Delicious | 4 - 5 leaves | 1 | JIP |
| Rice | RC | Ishikari | 2 - 2½ leaves | 4 | JIP |
| Tomato | TO | Ailsa Craig | 2 - 2½ leaves | 1 | PEAT |

JIP* = John Innes Potting Compost

TABLE V

| SPECIES | COMPOUND NO. | R | G | A | T | I |
|---|---|---|---|---|---|---|
| WW | 2 | | | | | |
| | 10 | 2 | 1 | | | 3 |
| | 11 | 2 | 1 | | 3 | 3 |
| | 12 | 2 | 1 | | 1 | 3 |
| | 13 | 3 | 2 | | 3 | 3 |
| BR | 2 | | | | | |
| | 10 | 1 | | | | |
| | 11 | 2 | 1 | | 1 | 2 |
| | 12 | 2 | 1 | | 1 | 2 |
| | 13 | 2 | 1 | | 1 | 2 |
| RC | 2 | | | | | |
| | 10 | 1 | 1 | | | 1 |
| | 11 | 2 | 2 | | 2 | 2 |
| | 12 | 3 | 1 | | 3 | 2 |
| | 13 | 2 | 1 | | 3 | 2 |
| AP | 2 | 2 | | | | 1 |
| | 10 | 1 | 1 | | | 1 |
| | 11 | 1 | 1 | | | 1 |
| | 12 | 1 | | | | 1 |
| | 13 | 2 | | | | 2 |

TABLE V (CONT/D)

| SPECIES | COMPOUND NO. | R | G | A | T | I |
|---|---|---|---|---|---|---|
| MZ | 10 | | | | | |
| | 11 | | | | | |
| | 12 | | | 1 | | |
| | 13 | | | | | |
| TO | 10 | 2 | 1 | | | |
| | 11 | 2 | 2 | | | |
| | 12 | 2 | 2 | | | 1 |
| | 13 | | | | | |

Key :

R = Retardation
G = Greening effect
A = Apical damage
T = Tillering or side shooting
I = Interligular or internodal length reduction All effects are scored visually on a 1-3 basis where

1 = 10-30%
2 = 31-60%
3 = 61-100% Blank means less than 10% effect.

EXAMPLE 15

Intermediate Retardant Test

Methodology

Three species are involved in this test RICE, SPRING-BARLEY and APPLES. The variety and growth stage at spray are outlined in Table VI. Compounds were applied at 500 ppm and 2000 ppm respectively (0.5kg and 2kg ha⁻¹ at a field volume of 10001 ha⁻¹ ) as an overall spray to rice and barley. This gives a foliar and root component in the test, i.e. this test will detect the activity of both root and foliar acting

compounds. The apples were either treated in the same manner as the cereals or the compounds were applied as 2000g ha˙ root drench and 2000g ha˙ overall plant spray. The rice was grown in 4" 'paddy' pots, i.e. the roots and bottom of the stems are immersed in water under conditions corresponding to those in paddy fields. Spring barley and apples were grown in 4" pots. The plants were assessed for height to top-most ligule at approximately 28 days after treatment for spring barley and rice and for height to apex between 14 and 28 days after treatment for apples (depending on time of year and growth stage). The results are presented in Tables VII to IX. In each case the result for each compound is compared to the height of the formulation blank in that test and presented as a percentage reduction in height compared to the formulation blank.

## TABLE VI

### PLANT MATERIAL FOR INTERMEDIATE RETARDANT TEST

| SPECIES | VARIETY | GROWTH STAGE AT TREATMENT | NO. PLANTS PER 4" POT | COMPOST TYPE |
|---|---|---|---|---|
| Spring Barley | Atem | 3 leaves | 4 | JIP 1 |
| Rice | Ishikari | 3 - 4 leaves | 2 | SM2 : JIP 1 |
| Apples | Red Delicious | 5 - 10 cm high | 1 | SM2 : JIP 1 |

JIP 1 = John Innes Potting Compost

SM 2 = a mixture of loam and grit

## TABLE VII - BARLEY

% HEIGHT REDUCTION FROM FORMULATION BLANK

| COMPOUND NO. | 500 g/ha | 2000 g/ha |
|---|---|---|
| 1 | 4 | 15 |
| 2 | 2 | 15 |
| 3 | NOT TESTED | 19 |
| 4 | 0 | 8 |
| 5 | 6 | 7 |
| 6 | 2 | 2 |
| 7 | 0 | 2 |
| 8 | 4 | 9 |
| 9 | 12 | 25 |
| 10 | 0 | 8 |
| 11 | 6 | 12 |
| 12 | 11 | 19 |
| 13 | 6 | 15 |

## TABLE VII - RICE

% HEIGHT REDUCTION FROM FORMULATION BLANK

| COMPOUND NO. | 500 g/ha | 2000 g/ha |
|---|---|---|
| 1 | 21 | 38 |
| 2 | 5 | 17 |
| 3 | NOT TESTED | 17 |
| 4 | 12 | 8 |
| 5 | 24 | 40 |
| 6 | 20 | 37 |
| 7 | 7 | 11 |
| 8 | 10 | 21 |
| 9 | 9 | 19 |
| 10 | 11 | 13 |
| 11 | 16 | 20 |
| 12 | 24 | 37 |
| 13 | 7 | 16 |

TABLE IX - APPLE

| COMPOUND NO. | FOLIAR ONLY APPLICATION | | FOLIAR SPRAY | ROOT DRENCH |
|---|---|---|---|---|
| | 500 $g/ha^{-1}$ | 2000 $g/ha^{-1}$ | 2000 $g/ha^{-1}$ | 2000 $g/ha^{-1}$ |
| 1 | 0 | 0 | - | - |
| 2 | - | - | 28 | 5 |
| 3 | - | - | - | 1 |
| 4 | 0 | 0 | - | - |
| 5 | 0 | 15 | - | - |
| 6 | 0 | 12 | - | - |
| 7 | 7 | 21 | - | - |
| 8 | 0 | 20 | - | - |
| 9 | - | - | 28 | 5 |
| 10 | - | - | 0 | 0 |
| 11 | - | - | (1000g) 0 | 28 |
| 12 | - | - | (1000g) 5 | 12 |
| 13 | - | - | 4 | 0 |

The manner in which the compounds of the present invention may be formulated into compositions suitable for application is shown generally in the following indicative illustrations numbered as Examples 16 to 25.

EXAMPLE 16

An emulsifiable concentrate is made up by mixing the following ingredients. and stirring the mixture until all the constituents were dissolved.

Compound of Table I 10%
Calcium dodecylbenzensulphate 5%
"SYNPERONIC" NP13 5%
"Aromasol" H 80%

EXAMPLE 17

A composition in the form of grains readily dispersible in a liquid, eg. water. is prepared by grinding together the first three ingredients in the presence of added water and then mixing in the sodium acetate. The resultant mixture is dried and passed through a British Standard mesh sieve, size 44-100, to obtain the desired size of grains.

Compound of Table I 50%
"Dispersol" T 25%
'SYNPERONIC" NP5 1.5%
Sodium acetate 23.5%

EXAMPLE 18

The following ingredients are ground together to produce a powder formulation readily dispersible in liquids.

Compound of Table I 45%
"Dispersol" T 5%
"SYNPERONIC" NX 0.5%
"Cellofas" B600 2%
China clay GTY powder 47.5%

EXAMPLE 19

The active ingredient is dissolved in acetone and the resultant liquid is sprayed on to the granules of attapulgite clay. The solvent is then allowed to evaporate to produce a granular composition.

Compound of Table I 5%
Attapulgite granules 95%

EXAMPLE 20

A composition suitable for use as a seed dressing is prepared by mixing the three ingredients.

Compound of Table I 50%
Mineral oil 2%
China clay 48%

EXAMPLE 21

A dusting powder is prepared by mixing the active ingredient with talc.

Compound of Table I 5%
Talc 95%

EXAMPLE 22

A flowable formulation is prepared by bead-milling the constituents set out below and then forming an aqueous suspension of the ground mixture with water.

Compound of Table I 40%
"Dispersol" T 4%
"SYNPERONIC" NP5 1%
Water 55%

EXAMPLE 23

A dispersible powder formulation is made by mixing together the ingredients set out below and then grinding the mixture until all were thoroughly mixed.

Compound of Table I 25%
"Aerosol" OT/B 2%
"Dispersol" A.C. 5%
China clay 28%
Silica 40%

EXAMPLE 24

This Example illustrates the preparation of a dispersible powder formulation. The ingredients are mixed and the mixture then ground in a comminution mill.

Compound of Table I 25%
"PERMINAL" BX 1%
"Dispersol" T 5%
Polyvinylpyrrolidone 10%
Silica 25%
China clay 34%

EXAMPLE 25

The ingredients set out below are formulated into dispersible powder by mixing then grinding the ingredients.

Compound of Table I 25%
"Aerosol" OT/B 2%
"Dispersol" A 5%
China clay 68%

In Examples 11 to 20 the proportions of the ingredients given are by weight.

There now follows an explanation of the compositions or substances represented by the various Trade Marks and Trade Names mentioned above.

"SYNPERONIC" NP13 : a condensate of nonyl phenol (1 mole) with ethylene oxide (13 moles).
"AROMASOL" H : a solvent mixture of alkylbenzenes.
"DISPERSOL" T AND AC : a mixture of sodium sulphate and a condensate of formaldehyde with sodium naphthalene sulphonate.
"SYNPERONIC" NP5 a condensate of nonyl phenol (1 mole) with naphthalene oxide (5.5 moles).
CELLOFAS B600 : a sodium carboxymethyl cellulose thickener.

**Claims**

1. A thiophene, furan or tetrahydrofuran triazolyl derivative having the general formula (I) or (II) :

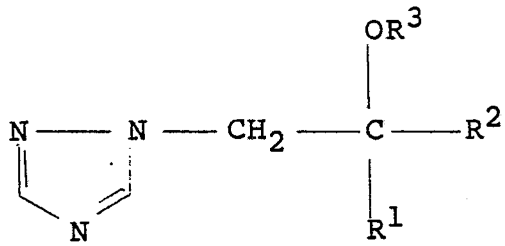

(I)

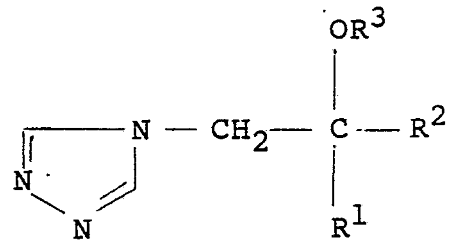

(II)

and stereoisomers thereof, wherein $R^1$ is tertiary butyl which may be optionally substituted with one or more halogen atoms; $R^2$ is the group : $-(CH_2)_n-Z$
or $-CH=CH-X$
or $-C\equiv C-Z$
where n is an integer from 0 to 3 and Z is an optionally substituted thiophene, furan, or tetrahydrofuran ring; and X is an optionally substituted thiophene or tetrahydrofuran ring; and $R^3$ is hydrogen or an alkyl group containing from 1 to 4 carbon atoms; and salts, esters and metal complexes of the compound of formula (I) or (II) wherein $R^3$ is hydrogen.

2. A triazolyl derivative according to claim 1 wherein the optional substituents in the group Z or X respectively are; alkyl containing from 1 to 6 carbon atoms; cycloalkyl containing from 3 to 6 carbon atoms; alkylcycloalkyl containing a total of from 4 to 6 carbon atoms; cycloalkylalkyl containing a total of from 4 to 6 carbon atoms; alkenyl containing from 2 to 6 carbon atoms; alkynyl containing from 2 to 6 carbon atoms; and halogen.

3. A triazolyl derivative according to claim 2 wherein Z or X is optionally substituted with alkyl containing from 1 to 4 carbon atoms.

4. A triazolyl derivative according to any of the preceding claims wherein $R^1$ is tertiary-butyl.

5. A triazolyl derivative according to any of the preceding claims wherein $R^3$ is hydrogen or methyl.

6. A triazolyl derivative according to any of the preceding claims wherein n is 0, 1 or 2.

7. A process for preparing a compound of general formula (I) as defined in claim 1 which comprises reacting a compound of general formula (IIIa) or (IIIb) :

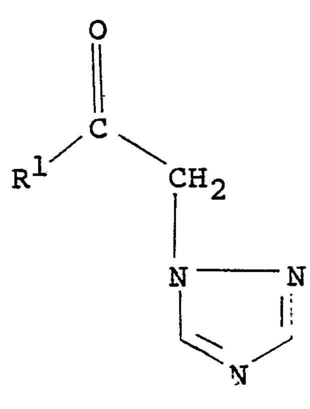

(IIIa)

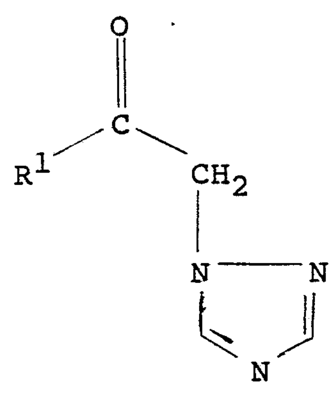

(IIIb)

where $R^1$ and $R^2$ are defined in claim 1 with an organometallic compound which may be represented by the general formula (IVa) or (IVb) :

$R^1M$ (IVa)
$R^2M$ (IVb)

where M is a suitable metal and optionally, reacting the product with the appropriate halide, acid chloride or acid anhydride in the presence of a suitable base or with the appropriate acid or metal complexing agent to form the compound of formula (I) wherein $R^3$ is an alkyl group is defined in claim 1 or to form the salt, ester or metal complex of the compound of formula (I) wherein $R^3$ is hydrogen.

8. A process for preparing a compound of general formula (I) as defined in claim 1 wherein $R^3$ is hydrogen and $R^2$ is $-CH_2-Z$ which comprises reacting a compound of general formula (VII) :

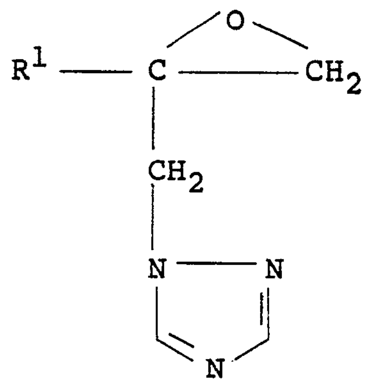

(VII)

with an organometallic compound of general formula Z-M where M is a suitable metal and optionally, reacting the product with the appropriate halide, acid chloride or acid anhydride in the presence of a suitable base or with the appropriate acid or metal complexing agent to form the compound of formula (I) wherein $R^3$ is an alkyl group is defined in claim 1 or to form the salt, ester or metal complex of the compound of formula (I) wherein $R^3$ is hydrogen.

9. A process for preparing a compound of general formula (I) or (II) wherein $R^3$ is hydrogen which comprises reacting a compound of general formula (V) or (VI) :

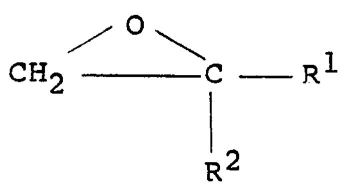

(V)

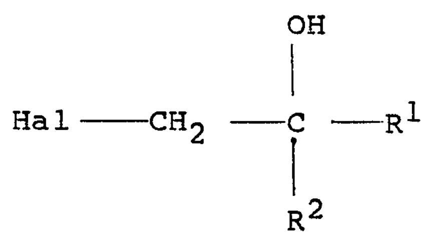

(VI)

wherein $R^1$ and $R^2$ are as defined and Hal is a halogen atom with 1,2,4-triazole in the form of one of its alkali metal salts in the presence of an acid binding agent and optionally, reacting the product with the appropriate halide, acid chloride or acid anhydride in the presence of a suitable base to form the compound of formula (I) or (II) wherein $R^3$ is an alkyl group is defined in claim 1 or to form the salt, ester or metal complex of the compound of formula (I) or (II) wherein $R^3$ is hydrogen.

10. A process for preparing a compound of formula (I) or (II) as defined in claim 1 wherein $R^2$ is the group $-(CH_2)_2-Z$ which comprises reduction of the corresponding $-CH=CH-X$ or $-C\equiv C-Z$ respectively.

11. A plant growth regulating composition comprising a plant growth regulating amount of a triazolyl derivative according to claim 1 and an agrochemically acceptable carrier or diluent.

12. A method of regulating plant growth which comprises applying to the plant, to the seed of the plant, or to the locus of the plant or seed, a plant growth regulating amount of a triazolyl derivative according to claim 1.

European Patent Office

# EUROPEAN SEARCH REPORT

Application number

EP 87311390.6

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| X | EP - A2 - 0 052 424 (JCJ)<br>* Claims 1,7,11,12 * | 1,7,8, 9,11 | C 07 D 405/06<br>C 07 D 409/06<br>A 01 N 43/64 |
| A | EP - A2 - 0 071 568 (CIBA-GEIGY)<br>* Examples 1.73, 1.112, 2.46, 2.55, 2.56, 2.79, 2.88; abstract * | 1,11 | |
| A | EP - A1 - 0 107 392 (PFIZER)<br>* Example 3 * | 1 | |
| A | DE - A1 - 3 321 158 (BAYER)<br>* Claims 1,8,9 * | 1,11 | |
| | | | TECHNICAL FIELDS SEARCHED (Int Cl 4) |
| | | | C 07 D 405/00<br>C 07 D 409/00 |

The present search report has been drawn up for all claims

| Place of search | Date of completion of the search | Examiner |
|---|---|---|
| VIENNA | 24-02-1988 | HAMMER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document